(19) Europäisches Patentamt
European Patent Office
Office européen des brevets

(11) **EP 4 643 832 A1**

(12) **EUROPEAN PATENT APPLICATION**

(43) Date of publication:
**05.11.2025 Bulletin 2025/45**

(21) Application number: **24173166.0**

(22) Date of filing: **29.04.2024**

(51) International Patent Classification (IPC):
**A61F 13/15** (2006.01) **A61F 13/494** (2006.01)
**A61F 13/511** (2006.01) **A61F 13/512** (2006.01)
**A61F 13/53** (2006.01)

(52) Cooperative Patent Classification (CPC):
**A61F 13/513; A61F 13/15203; A61F 13/532;
A61F 13/537**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **The Procter & Gamble Company
Cincinnati, OH 45202 (US)**

(72) Inventors:
• **Ehrnsperger, Bruno Johannes**
**65824 Schwalbach am Taunus (DE)**
• **Hettinga, Johanna Karpagam**
**65824 Schwalbach am Taunus (DE)**
• **Kamphus, Juliane**
**65824 Schwalbach am Taunus (DE)**

(74) Representative: **P&G Patent Germany
Procter & Gamble Service GmbH
Sulzbacher Straße 40
65824 Schwalbach am Taunus (DE)**

(54) **ABSORBENT ARTICLE FOR LOW VISCOSITY FECAL MATTER**

(57) A personal absorbent article (20) comprising in this order a topsheet (24), an optional acquisition layer (50), an apertured central layer (60) and a backsheet (25). The apertured central layer may be a nonwoven comprising or consisting of superabsorbent fibers. The topsheet, together with the optional acquisition layer when present, at least partially conforms to the apertures of the apertured central layer so that a three-dimensional surface on the wearer-facing side of the article is provided.

**Fig. 2**

**Description**

FIELD OF THE INVENTION

The invention relates to personal hygiene absorbent articles, such as but not limited to diapers. The articles of the invention comprise an apertured central layer that is especially useful for absorbing and retaining runny BM (bowel movement).

BACKGROUND OF THE INVENTION

Absorbent articles for personal hygiene, such as diapers for babies or incontinent adults, are designed to absorb and contain body exudates. These absorbent articles typically comprise several layers, in particular a fluid permeable topsheet on the wearer-facing side, an acquisition layer underneath the topsheet, an absorbent core, and a fluid impermeable backsheet on the garment-facing side.

Babies under 6 months of age and exclusively breastfed babies typically have runny (relatively low viscosity) feces that create specific challenges for a quick absorption and leakage prevention.

It has been proposed to use three-dimensional protrusions at the surface of the topsheet to provide a surface topography that limits the spreading of runny BM, characteristic of young infants. These 3D topsheet comprise protrusions and/or depressions that can restrain the movement of runny stool. Apertures may be provided in these topsheets. Examples of such 3D topsheets are disclosed in US2017/0258649A1 (Rosati et al.). However, these topsheets are more costly, and skin can still remain in contact for a prolonged time with the soiled topsheet, which is not desirable.

Patterned apertured topsheet (PATS) have also been proposed. These nonwoven topsheet comprise numerous elongated apertures that let runny stools pass through into the article, where the stool can be absorbed by the absorbent core. These apertured topsheet may be unbonded to the underlying acquisition layer in order to create void volume. These are for example disclosed in WO2016/073712A1 (Arora at al.). It has also been proposed to use in combination with a patterned apertured topsheet a sublayer comprising through or blind holes to isolate feces away from the skin (WO2007/034453A1, Ponomarenko et al.). However, these apertured topsheets tend to be rigid, stick to the skin, and the apertures can mark the skin.

SUMMARY OF THE INVENTION

The present invention is for a personal absorbent article. In a first aspect, the absorbent article comprises, in this order, from its wearer-facing side to its garment-facing side: a topsheet, an optional acquisition layer, an apertured central layer and a backsheet. The topsheet is preferably a non-apertured nonwoven. The topsheet, together with the optional acquisition layer when present, is conformable and can at least partially conform to the apertures of the central layer. This provides a three-dimensional surface on the wearer-facing side of the article in use when the baby sits on the topsheet. The three-dimensional pattern can be described as comprising hills- and-valleys, with the valleys corresponding to the apertures of the central layer and the hills corresponding to the portions of the central layer between the apertures. Various other aspects of the invention are indicated in the attached claims, including preferred but non-limiting features in the dependent claims. The following are non-limiting, but advantageous features of the invention.

The topsheet, together with the acquisition layer if present, may have together a Horizontal Bending Drop Test at 100 mm (HBD@100mm) of at least 60 mm (the test is disclosed in the Test section below).

The apertured central layer may have a % Effective Open Area in the range of from 10% to 50%, as measured on the topsheet-facing side of the apertured central layer using the Aperture Dimensions Test described herein.

At least some and preferably all the apertures of the central layer may have an Effective Aperture Area in the range of from about 2 mm$^2$ to 500 mm$^2$, as measured on the topsheet-facing side of the apertured central layer by the Aperture Dimensions Test described herein.

The apertures of the central layer may be formed by mechanically perforating a material web such as a nonwoven web. However, the central layer may also be advantageously formed by other means that avoid scrap, such as slit-and-stretch process, where slits are cut and then the layer stretched in a direction different from the direction of the slits, or by airlaying fibers on a three-dimensional mold for example.

The apertured central layer is preferably absorbent so that it can absorb urine and withdraw moisture from the runny stools. In particular, the central layer may be a nonwoven comprising or consisting of superabsorbent fibers.

The article may comprise an additional layer between the apertured central layer and the backsheet. This additional layer may be another apertured central layer, which may be the same as the first layer in order to double the thickness of the first apertured central layer, or a different layer comprising different material, in particular a supplementary absorbent layer, which may be apertured or non-apertured. Various absorbent layers may be used, the secondary absorbent layer may for example comprise cellulose fibers and/or superabsorbent particles such as an absorbent layer comprising a mixture of cellulose fibers and superabsorbent particles or an absorbent layer comprising superabsorbent particles and/or superabsorbent fibers which is free of cellulose fibers.

These and further aspects of the inventions are described herein below in more details.

BRIEF DESCRIPTION OF THE DRAWINGS

Fig. 1 shows a schematic view of a taped diaper according to an embodiment of the invention;
Fig. 2 shows a cross-section of the diaper of Fig. 1;
Fig. 3 shows an alternative central layer comprising two apertured central sublayers;
Fig. 4 shows an apertured central layer and a supplementary absorbent layer;
Fig. 5 shows an apertured central layer and a different supplementary absorbent layer;
Fig. 6 shows an apertured central layer and a different supplementary absorbent layer;
Fig. 7 shows a photograph of an exemplary apertured central layer in isolation;
Fig. 8A, 8B shows stain appearance of an inventive example and comparative market product, respectively;
Fig. 9 shows a schematic setup for conducting the Horizontal Bending Drop Test.

DETAILED DESCRIPTION OF THE INVENTION

**Absorbent article**

As used herein, "absorbent articles" refers to personal hygiene devices that are placed on the crotch of a wearer to absorb and contain body exudates. Baby care articles are products intended for babies, toddlers and/or children, relating to disposable absorbent articles including taped diapers, pant diapers, absorbent inserts. Feminine care articles are products relating to catamenial pads, incontinence pads, interlabial pads, panty liners, pessaries, sanitary napkins. Adult incontinence articles are products intended for adults, relating to disposable absorbent articles including taped and pant diapers, absorbent inserts, incontinence pads, panty liners. While the invention is especially useful for baby care articles, it may also be used in feminine care articles or adult incontinence articles. The absorbent articles of the invention are typically disposable and are preferably recyclable.

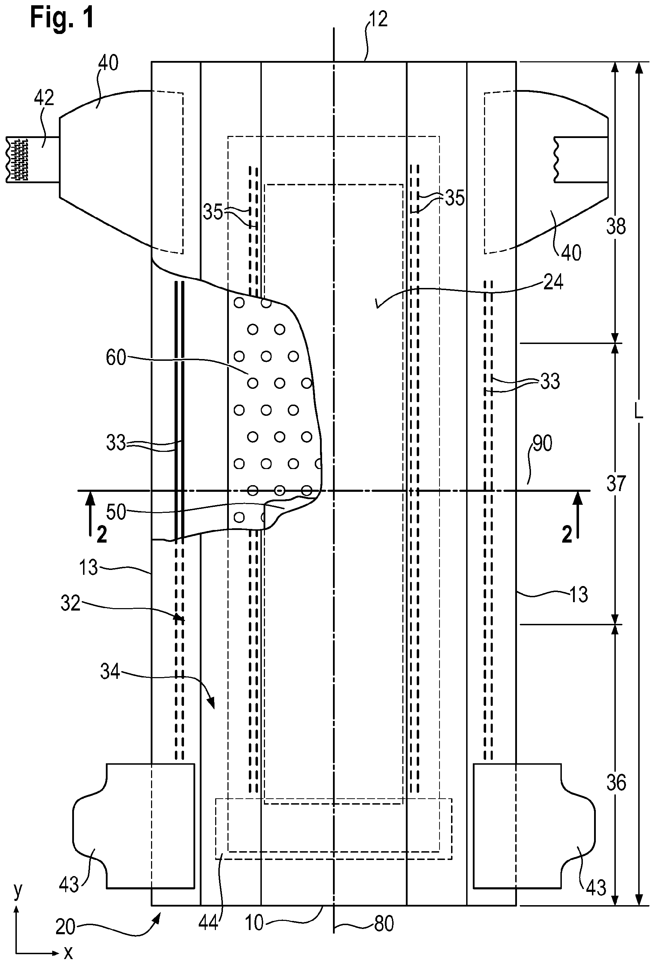

As used herein, "diapers" refers to absorbent articles generally worn by babies, infants and incontinent adults about the lower torso so as to encircle the waist and legs of the wearer and that is specifically adapted to receive and contain urinary and fecal waste. Diapers are typically proposed as taped diapers or pant diapers. Taped diapers have a fastening system (as illustrated in Fig. 1 for example), where the waist opening and leg openings are formed when the diaper is applied onto the wearer by releasably attaching the longitudinal edges of the front waist region and back waist region to each other. In pant diapers, on the other hand the longitudinal edges of the waist regions are attached to each other to form a pre-formed waist opening and leg openings. A pant diaper is placed in position on the wearer by inserting the wearer's legs into the leg openings and sliding the pant diaper into position about the wearer's lower torso. A pant may be pre-formed by any suitable techniques including, but not limited to, joining together portions of the absorbent article using re-fastenable and/or non-refastenable bonds (e.g., seam, weld, adhesive, cohesive bond, fastener, etc.). A pant may be pre-formed anywhere along the circumference of the article (e.g., side fastened, front waist fastened).

An exemplary, non-limiting absorbent article according to the invention is discussed herein in relation to Figures 1-2. Figure 1 is a schematic view of the exemplary diaper 20, in a flat-out state, with portions of the diaper being cut-away showing the different layers of the diaper. This diaper 20 is shown for illustration purpose only, as the present invention may be embodied in a wide variety of diapers or other absorbent articles, such as pant diapers having pre-formed side seams. The side seams of a pant article can be opened by cutting or otherwise, if it is desired to place the pant in a flattened-out configuration similar to Fig. 1.

As illustrated in Fig. 1, the absorbent article, whether a taped diaper or a pant diaper, can be notionally divided in a front waist region 36, a back waist region 38 opposed to the first waist region 36, and a crotch region 37 located between the front waist region 36 and the back waist region 38. The crotch region, the front waist region and the back waist region are hereby defined as each delimiting one third of the length of the absorbent article along the longitudinal centerline 80. The longitudinal centerline 80 is the imaginary line separating the diaper along its length in two equal right and left halves. The transversal centerline 90 is the imaginary line perpendicular to the longitudinal centerline 80 in the plane of the flattened-out diaper and going through the middle of the length of the diaper. The periphery of the diaper 20 is defined by the outer edges of the diaper. The longitudinal edges 13 of the diaper may run generally parallel to the longitudinal centerline 80 of the diaper 20 and the front waist edge 10 and the back waist edge 12 typically run generally parallel to the transversal centerline 90 of the diaper 20. However, these article edges do not need to be straight, as they may be curved to better fit the wearer.

As illustrated in Figures 1-2, the absorbent article comprises in this order a topsheet 24 on its wearer-facing side, an optional acquisition layer 50, an apertured central layer 60 and a backsheet 25 on its garment-facing side, which are discussed in more details below.

The absorbent article 20 may also comprise a pair of inner barrier leg cuffs 34 and a pair of outer leg cuffs 32, as is known in the art. The inner barrier cuffs 34 can extend upwards from the surface of the article to provide retention of the waste, while the outer cuffs are typically formed in the plane of the chassis of the article as defined by topsheet and backsheet. These cuffs are preferably elasticized, as is known in the art, for example using elastic threads 33, 35 as represented in the Figures 1-2. The cuffs may be comprised of a nonwoven material having barrier properties as is known in the art. While not illustrated, the absorbent article may also be in the form of a pant diaper comprising an inner and outer belt nonwovens as is known in the art.

The absorbent article may comprise a fastening system, such as hook-and-loop fastening member as is known in the art. Such system typically comprises a pair of tape tabs 42 disposed on the back ears 40, such as adhesive tape tabs or tape tabs comprising the hook elements, and a landing zone 44 disposed on the front external surface of the diaper (e.g. a nonwoven web providing the loops in a hook-and-loop fastening system).

The front and/or back ears may be separate components attached to the absorbent article as represented in Fig. 1, or may alternatively be formed from portions of the topsheet and/or backsheet that extend transversally such that these portions form all or a part of the front and/or back ears 40, 43. Also combinations of the aforementioned are possible, such that the front ears 43 and/or back ears 40 are formed by portions of the topsheet and/or backsheet while additional materials are attached to form the overall front and/or back ears 40, 43. The front and/or back ears may be elastic or non-elastic. Also, the front ears 40 may be applied as separate components attached to the absorbent article while the back ears (or parts thereof) may be continuous with portions of the backsheet and/or topsheet - or vice versa. While taped diapers typically comprise back ears 40, and front ears 43, these are typically not present in pant-type absorbent articles having pre-formed side seams.

The absorbent article may also comprise other optional but conventional elements, which are not represented for simplicity, such as a back waist elastic feature, a front waist elastic feature, a lotion applied onto the wearer-facing surface of the topsheet, or a urine indicator disposed on the inner side of the backsheet that changes color when in contact with urine.

The topsheet 24, the backsheet 25, and the central layer 60 and other layers of the absorbent article may be assembled in a variety of well-known configurations, such as by gluing, heat embossing, ultrasonic bonding or combinations thereof. Exemplary diaper configurations are described generally in US3,860,003; US5,221,274; US5,554,145; US5,569,234; US5,580,411; and US6,004,306.

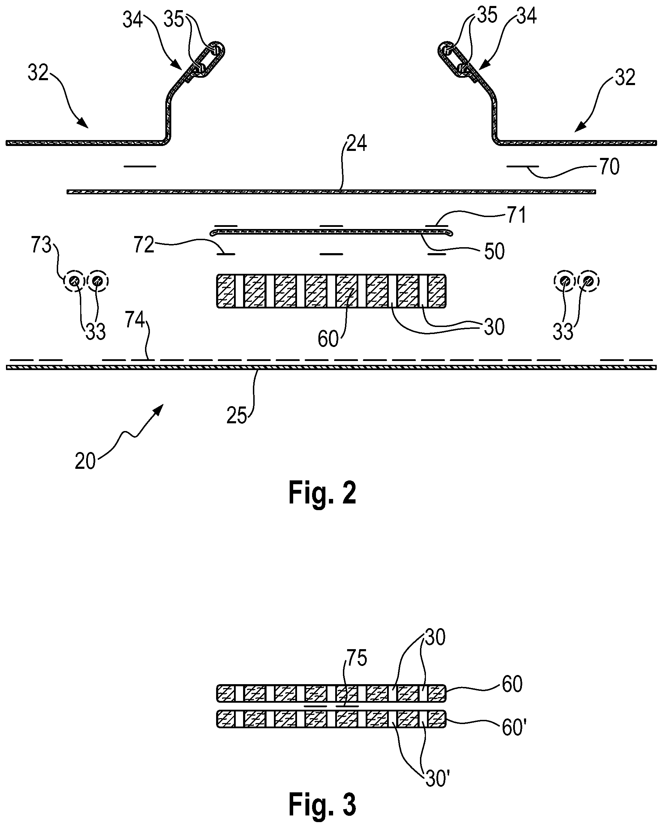

Some of these bonds are indicated in dashed lines in Fig. 2. There may be a fusion bond 70 between the barrier cuff material 32-34 and the topsheet 24. The rest of the bonds shown may be typically adhesive bonds. The elastics 33 are typically coated with an elastic adhesive 73 to anchor these in the chassis of the diaper, a backsheet adhesive 74 may be spiral applied on the backsheet.

The acquisition layer 50, when present, and topsheet layer 24 may be at least partially bonded to form a topsheet-acquisition layer laminate. The acquisition layer 50 may be adhesively bonded by an adhesive, such as slot coated adhesive 71, to the topsheet 24. The apertured central layer 60 is advantageously only partially attached to the overlying layer (topsheet or acquisition layer) so that the overlying layer can more easily conform to the apertured layer, in particular more easily penetrate the apertures of the central layer. Thus, the upper surface of the apertured central layer 60 may be bonded over from 5% to 40% of its surface with the overlying layer (e.g. acquisition layer). For example, as represented in Fig. 2, there may be three longitudinally-extending adhesive slots 72 attaching the acquisition layer 50 and the central layer 60, each slot covering about 5% of the surface of the apertured central layer. Of course, other executions are possible.

## Topsheet

The topsheet 24 forms the wearer-facing surface of the article that is in intimate contact with the skin of the wearer, in particular the perineal area. At least a portion of and typically all of the topsheet is liquid permeable, permitting liquid bodily exudates to readily penetrate through its thickness. The topsheet is preferably a nonwoven and is preferably non apertured. Any conventional non-apertured nonwoven topsheet may be typically suitable in the present invention. It is believed that non-apertured topsheet are less prone to stick to a baby's skin compared to apertured topsheet, which are more prone to stick to the skin.

Typically, a nonwoven topsheet may have a basis weight in the range of 10 gsm to 30 gsm. The nonwoven topsheet may also be hydrophilized, in particular it may comprise a surfactant agent that has been coated or otherwise applied on the nonwoven.

Nonwovens are sheet or web structures bonded together by entangling fibers or filaments mechanically, thermally, or chemically. They are flat, porous sheets that are made directly from separate fibers. They are not made by weaving or knitting and do not require converting the fibers to yarn. Common process to make nonwovens are meltblowing, spunbonding, solvent spinning, electrospinning, carding and airlaying. The basis weight of nonwoven webs is usually expressed in grams per square meter (g/m$^2$ or gsm).

Spunlaid nonwovens are made in one continuous process where molten polymer granules are extruded into filaments through so called spinnerets. The continuous filaments are stretched and quenched before being deposited on a conveyor belt to form a uniform web. Spunlaid nonwovens have an increased strength compared to carded nonwovens, due to the attenuation of the filaments. The term "spunbonded nonwoven" designates thermo bonded spunlaid.

Carded nonwovens on the other hand are made from staple fibers that are mechanically processed. Staple fibers are fibers having a discrete length which is much smaller than spunlaid fibers. In a typical carding process, bales of staple fibers are brought to a carding line where the bales are unwrapped. The bales are opened at a bale opener and the fibers are initially separated and transported by conveyer belt to the fiber preparation step where further fiber opening is taking place. The staple fibers then enter a master chute where the web going into the card is prepared. The master chute is also controlling the dosing of the fibers and ensures that there is an even distribution of fibers throughout the width of the web. Inside the card, the fibers are distributed in both the machine direction, MD, and cross direction, CD, depending on several parameters such as line speed, fiber cohesion and randomizers amongst other. The carded web obtained has limited initial strength and needs therefore to be consolidated. Consolidation is typically carried out by air-through bonding or calendering bonding.

Air-through bonding (ATB) is a type of thermal bonding that uses a hot air stream to melt bicomponent fibers mixed with the staple fibers. The bicomponent fibers typically comprise Polyethylene (PE) in the sheath and Polypropylene (PP) in the core. PE has a lower melting temperature and junction points can thus be created by fusing the PE component fibers of individual component fibers, including PE mono-fibers. Air-through bonded carded nonwovens typically have excellent softness. ATB is however a comparably costly approach as it requires relatively expensive bicomponent fibers and uses a high energy consumption at a comparably low line speed.

Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers.

The invention advantageously comprises an acquisition layer between the topsheet and the apertured central layer. The topsheet and the optional acquisition layer when present are conformable and can follow the shape of the apertured central layer by at least partially penetrating the apertures 30 of the central layer. In this way the topsheet can form a three-dimensional surface on the wearer-facing side of the article, especially when the topsheet has been subjected to a runny fecal matter.

The conformability of the topsheet itself when used alone, or when in combination with an acquisition layer, may be easily measured using the Horizontal Bending Drop Test at 100 mm (HBD@100mm), which is disclosed in the Test section below. In short, this test measures the vertical drop of a piece of material or laminate which is 100 mm long. The topsheet (together with the acquisition layer if present) preferably have a HBD@100mm value of at least 55 mm, and preferably at least 60 mm, or at least 65 mm, or at least 70 mm, as measured with the Horizontal Bending Drop at 100 mm Measurement Method described herein.

## Acquisition layer 50

The absorbent articles of the invention may advantageously further comprise an acquisition layer 50 between the topsheet 24 and the central layer 60. An acquisition layer is especially useful wherein the topsheet is not apertured. The acquisition layer is in close contact with the topsheet and can extract the moisture from the runny BM away from the skin, thus providing for an improved skin comfort.

The acquisition layer typically comprises a single layer, but it is not excluded that the acquisition layer may also comprise more than one layer. However, when the acquisition layer comprises more than one layer, the laminates may become too stiff and less conformable.

Any conventional materials having fluid acquisition properties, integrity and conformability may also be used as acquisition layer. The acquisition layer advantageously comprises or consists of a non-apertured nonwoven having a basis weight in the range of from 20 gsm to 80 gsm.

An example of acquisition layer 50 may be a surfactant treated, latex bonded, nonwoven acquisition layer.

As another example, the acquisition layer 50 that may be used in the present invention may also be a spunlace layer. Spunlace are nonwovens comprising absorbent fibers, stiffening fibers and resilient fibers, as for example disclosed in WO2020/205485 (Peri et al.). Such spunlace layer may typically comprise from about 20 percent to about 75 percent of absorbent fibers, from about 1 percent to about 50 percent of stiffening fibers, and from about 10 percent to about 50 percent of resilient fibers.

The drapability of conformity of the topsheet-acquisition layer laminate (or topsheet alone when there is no acquisition layer) should be relatively high for the present invention, so that the laminate can form a three-dimensional surface following the topology of the apertures in the central layer.

The properties of the topsheet or topsheet-acquisition layer laminate may be measured by different methods, in particular the Horizontal Bending Drop Test at 100 mm (HBD@100mm) and the Handle-o-meter Test Method (the test methods are as described below).

The Horizontal Bending Drop Test at 100 mm (HBD@100mm) measures the drapability of the topsheet-acquisition layer laminate (or topsheet alone when no acquisition layer present). The laminate (or topsheet) should preferably have a HBD@100mm value of at least 55 mm, or at least 60 mm, or at least 65 mm, typically up to 90 mm, as measured with the Horizontal Bending Drop at 100 mm Measurement Method described herein.

The Handle-o-meter test method can be used to measure the stiffness of the topsheet-acquisition layer laminate (or topsheet alone if used without an acquisition layer). The stiffness as measured by this method may advantageously be under 90 g, in particular in the range of from 10 g to 80 g.

## Apertured central layer 60

The absorbent article 20 of the invention comprises a central layer 60 comprising a plurality of apertures 30. The central layer 60 may be disposed directly under the topsheet if there is no acquisition layer. However, when the topsheet is not apertured, the article advantageously comprises an acquisition layer and thus the apertured central layer is typically disposed between a topsheet-acquisition layer laminate and the backsheet. The apertured central layer comprises a topsheet-facing side oriented towards the topsheet and a backsheet-side oriented towards the backsheet. The apertured central layer 60 preferably has absorbent properties. The apertured central layer may thus replace a conventional absorbent core in the present invention.

The wearer-facing side of the central layer 60 comprises a plurality of apertures 30 which are sufficiently large so that the topsheet-acquisition layer laminate (or topsheet alone if no acquisition layer is present) can conform to apertures in the central layer, so that the surface of the topsheet is provided with a "hills and valleys" pattern. Thus, the term "apertures" as used herein does not encompass interspatial space or pores which are formed between fibers when fibers are deposited or assembled in an airlaid or nonwoven layer. Rather, the apertures of the central layer in the invention are macroscopic, intentionally formed and disposed in pre-defined pattern, as will be described further below.

The central layer may be comprised of any suitable material that has sufficient integrity to comprise apertures, in particular a nonwoven. As discussed below, the central layer may be a nonwoven comprising or consisting of superabsorbent fibers (SAF) which is apertured.

The apertures may be obtained by any suitable means. As discussed in the example, the apertures may be obtained by perforating the material of the central layer using a punching tool having a series of spikes disposed in a pattern. The apertures in such a method may be typically circular. However, a punching method may be wasteful and create scrap when the punched-out material cannot be easily recycled. A more economic method for creating apertures at industrial scale is the slit and stretch method, which comprises the subsequent steps of cutting elongated slits in the material, typically the slits being parallel to the machine direction, and then stretching the material in another direction, typically orthogonally to the slits, i.e. in cross-direction, to open the slits. The obtained apertures may have generally diamond shaped apertures.

The apertures may typically have an Aspect Ratio in the range of from 1 to 10, in particular from 1 to 5, or from 1 to 3. The aspect ratio is calculated by dividing the length of an aperture by its width, as indicated in the Aperture Dimensions Test described below.

The apertures are preferably "through" apertures, extending from the top surface of the central layer to its bottom surface. The apertures thus preferably penetrate the whole thickness of the central layer ("through" apertures), but it is not excluded that some or all apertures may form blind holes formed on the topsheet-facing side and which are not extending through the whole of the thickness of the central layer.

The apertured central layer may have a % Effective Open Area in the range of from 10% to 50% as measured on the topsheet-facing side of the apertured central layer, as measured by the Aperture Dimensions Test described herein. If the apertures are not regularly disposed over the whole surface of the apertured central layer, then the % Effective Open Area is measured in the portion of the apertured central layer corresponding to the back region 36 and crotch region 37 of the article.

When the apertures are obtained by a punching out method using spikes, the individual apertures may be substantially circular having a diameter in the range of 2 mm to 20 mm. The individual apertures may be substantially identical, using the same spikes for each apertures but it is not excluded that the shape or diameter of the apertures may vary to provide a desired pattern, for example having differently shaped or dimensioned apertures in the front and/or back region of the diaper compared to the apertures in the crotch region.

The apertures may however have any other shapes, including irregular, square, rhombus and diamond shapes, especially when obtained by a process comprising the steps of slitting and stretching. When the apertures are diamond-shaped, their longest axis may have a length in the range of 2 mm to 20 mm, and the shortest axis may also have a length in the range of 2 mm to 20 mm.

More generally, the apertures of the central layer may thus comprise or consists of apertures having an Effective Aperture Area in the range of about 2 mm$^2$ to 500 mm$^2$, or 3 mm$^2$ to about 300 mm$^2$, in particular of from about 4 mm$^2$ to about 100 mm$^2$, or from about 5 mm$^2$ to about 50 mm$^2$. The Effective Aperture Area is measured by the Aperture Dimensions Test described herein.

For typical personal hygiene article applications, the central layer may typically comprise from 10 to 10,000 apertures, in particular from 50 to 2,000 apertures, in particular from 100 to 1,000 apertures.

The volume of the apertures can be approximated by multiplying the surface area of the apertures by the caliper of the central layer. This is the case when the apertures have substantially constant cross-section through the thickness of the central layer (e.g. circular or diamond cross-section, or any other regular shapes). The Effective Aperture Area as measured by the Aperture Dimensions Test as described herein can be used to determine the average surface area of the apertures. The caliper value for the central layer can be measured at a pressure of 0.85 kPa, as indicated further below in the Caliper and Density Measurement method below.

For example, an aperture having a 10 mm$^2$ surface area and a depth equal to the caliper of the central layer of e.g. 10 mm, has an individual volume of 100 mm$^3$ (as measured at a pressure of 0.85 kPa). More generally, the apertures may typically have an average individual volume in the range of 10 mm$^3$ to 2,000 mm$^3$, in particular from 20 mm$^3$ to 1,000 mm$^3$, wherein the caliper is measured at a pressure 0.85 kPa according to the Caliper and Density Measurement Method disclosed herein.

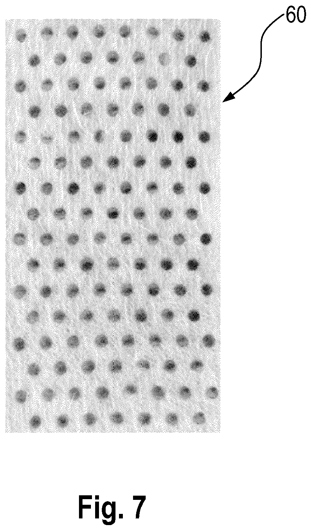

An exemplary aperture pattern consisting of a plurality of circular, punched-out apertures is shown in the photo of Fig. 7. The apertures may be arranged as a series of transversally-oriented rows, each row for example comprising from 5 to 10 apertures. However other patterns are of course possible, the apertures may for example be diagonally aligned or aligned longitudinally.

The % Effective Open Area and the Effective Aperture Area may be in most cases approximated from the specification of the perforating tool pattern or alternatively the slit and stretch parameters used to make the apertures. If the perforating tool and/or parameter are not directly available because the product is a third party produced product, these values can be measured using the Aperture Dimensions Test, which is disclosed herein below. While the measured values may be somewhat smaller values than the calculated "ideal" values due to the fibers being present at the edges of the apertures, the calculated values when available can be sufficient as a first approximation.

The basis weight of the apertured central layer may vary depending on various factors, such as the absorbency of the material, the presence or absence of an additional absorbent layer 62, and of course the intended usage (heavy adult incontinence applications may require more absorbency than for newborn babies). Typically, the basis weight of the apertured central layer may be in the range of from 10 gsm to 600 gsm. The central layer may be comprised of a single apertured layer, as illustrated in Fig. 2, however for practicality, the central layer may also be comprised of two (or more) superposed apertured layers 60, 60' of lower basis weight, as illustrated in Fig. 3. For example, a single 300 gsm SAF apertured nonwoven may be used as central layer, or a combination of two 150 gsm SAF apertured nonwoven sublayers where the apertures of one sub-layer are aligned with the apertures of the other sub-layer.

The apertured central layer 60 is advantageously a high loft layer, having a density in the range of from 0.01 g/cm$^3$ to 0.5 g/cm$^3$, in particular 0.02 g/cm$^3$ to 0.3 g/cm$^3$, as measured at a pressure of 0.85 kPa by the Caliper and Density Measurement Method disclosed below. Contributing to the high loft are the lofty nature of the nonwoven itself and the apertures therethrough.

The caliper of the apertured central layer 60 may also vary. For typical executions the caliper of apertured central layer 60 may be in the range of 1 mm to 20 mm, as measured at a pressure of 0.85 kPa by the Caliper and Density Measurement Method disclosed below. When the apertured central layer comprises two or more superposed apertured central layer 60, 60' as illustrated in Fig. 3, the caliper, basis weight and density are measured for the superposed layers.

The central layer 60 may be an absorbent layer comprising an absorbent material such as absorbent fibers and/or superabsorbent fibers. In one example, the central layer may be a nonwoven comprising or consisting of superabsorbent fibers.

The central layer 60 comprising or consisting of superabsorbent fibers may have a capacity of at least 7 g/g, or at least 8 g/g, or at least 10 g/g, or at least 15 g/g, or at least 18 g/g, or at least 20 g/g as measured according to the SAF Centrifuge Retention Capacity test set out herein.

The central layer 60 may preferably be a nonwoven comprising or consisting of superabsorbent fibers. Such a nonwoven is illustrated in Fig. 7, the apertures shown having a diameter of about 5 mm.

The central layer 60 may be rectangular as illustrated in the Figures, but other shapes are possible, such as a shaped layer having an hour-glass shaped, or tapered towards the back of the article. The apertures may be regularly disposed on the central layer 60, but it is also not excluded that the apertures distribution, shape or dimensions may vary across the surface of the central layer. In particular, the apertures density may be lower in the front region 36 of the article than in the back region 38 and/or crotch region 37 of the article, as the runny BM is more likely to be present in the back and crotch region of the article. Optionally, the front region of the article may be substantially free of apertures.

The central layer 60 may comprise a surfactant to increase the hydrophilicity of the central layer. The surfactant may be added during manufacture of the fibers of the central layer, such that the surfactant is distributed on the surface of the fibers as well as inside the fibers.

Alternatively, the central layer 60 may comprise a surfactant which has been added during manufacture of the central layer such that the surfactant is primarily distributed on the surface of the fibers of the central layer 60. The surfactant may be applied by any method known in the art, e.g. by spraying or dip-coating.

When the surfactant is applied as solution with a solvent, the solvent may be less than 50 weight-%, less than 40 weight-%, less than 30 weight-%, less than 20 weight-% based on the total amount of solution. The surfactant can be applied in an aqueous or organic solvent, for example sprayed at 0.5% wt. % as exemplified below for Hedipin® CFA-100.

The central layer 60 may comprise one or more surfactant(s) at a total level of 2% by weight of the central layer 60 or less, or less than 1%, or less than 0.6%, or less than 0.1% by weight of the central layer 60.

**Superabsorbent Fibers**

The term "Superabsorbent fiber" ("SAF") as used herein refers to a superabsorbent polymer material that is in a fibrous form. The superabsorbent fibers have a length and a cross-section, which may be the same or different for each superabsorbent fibers. The length is the largest dimension of the fiber when the fiber is or would be laid flat and straight on a surface, such that curves or crimps in the fiber disappear and the fiber becomes an approximately rod-like form. The cross-section is orthogonal to the length. For purposes herein, a fiber is a material that has a largest dimension and smallest dimension, wherein the largest dimension (also called the length) is at least 10 times, or at least 15 times, or at least 20 times the smallest dimension of the fiber (also called width). Preferably fibers are staple fibers and may have any cross-sectional shape such as circular, or substantially circular, cross-section.

Superabsorbent fibers are preferably made from the same materials as superabsorbent polymers, further described below. Typical superabsorbent materials are polyacrylate polymers, however it is not excluded that other polymer materials may also be used. For example, starch-based absorbent polymer material may also be used, as well as polyacrylamide copolymer, ethylene maleic anhydride copolymer, cross-linked carboxymethylcellulose, polyvinyl alcohol copolymers, crosslinked polyethylene oxide, and starch grafted copolymer of polyacrylonitrile.

Superabsorbent fibers have a high capacity to absorb fluids, such as urine and other bodily fluids. The capacity of superabsorbent fibers to absorb an aqueous 0.9% saline solution may be measured using the SAF Centrifuge Retention Capacity (SAF-CRC) test, which is described herein below. The superabsorbent fibers may have a capacity of at least 7 g/g, or at least 8 g/g, or at least 10 g/g, or at least 15 g/g, or at least 18 g/g, or at least 20 g/g as measured according to the SAF-CRC test set out herein.

The apertured central layer useful in the present invention may typically comprise from about 10% to about 100% by weight of superabsorbent fibers. The apertures central layer may comprise more than 20%, or more than 40%, or more than 60%, or more than 70%, or more than 90% by weight of superabsorbent fibers. The apertured central layer may comprise superabsorbent fibers in combination with from about 20% to about 80% of other synthetic fibers such as polyethylene, polypropylene, or bicomponent fibers or natural fibers such as cellulose fibers. Suitable bicomponent fibers include polyester/polypropylene, PE/PP, bicomponent fibers. The central layer of the invention may be in particular a nonwoven comprising from about 20% to about 100% by weight of superabsorbent fibers and from about 0% to about 80% by weight of bicomponent fibers.

Various patents have been published describing how to make suitable superabsorbent fibers such as spinning from an organic solvent (US3,983,095 US3,954,721), prepared from aqueous uncured but curable polymer compositions based on isobutylene-maleic anhydride copolymers. (US5,026,784, US5,151,465, US4,880,868, US4,743,244, US4,616,063, US 4,705,773, US4,731,067, US4,731,067, US4,743,244, US4,788,237 and US4,813,945).

More recently, a process was disclosed with wet spinning of a terpolymer of acrylic acid, methacrylic acid, and a crosslinker polymerized into the linear polymer at around 70°C, then extrusion at around 220°C leading to crosslinking and fiber drying, corresponding patents include US5,280,079, US5,147,956, US4,980,434, US4,962,172, US4,997,714 and US4,861,539.

Suitable nonwoven webs comprising superabsorbent fibers for use as the central layer of the present invention are commercially supplied by Technical Absorbents Ltd., Grimsby, UK (www.exploresaf.com). Alternatively, the central layer may be formed directly on the article production line, for example by depositing staple SAF fibers onto a continuous moving belt to provide an initial central layer which may be transported directly to the next processing step. Such a method of manufacturing absorbent fibrous structures is described in WO2016/191439 (Jackels).

Suitable nonwoven webs comprising SAF fibers that may be apertured and used in the central layer of the invention are further disclosed in US2022/0354713A1 (Ehrnsperger et al.).

When the central layer comprises or consists of SAF, the central layer 60 may have an amount of extractables of less than 20 weight-%, or less than 15 weight-%, or less than 12 weight-%, or less than 10 weight-% based on the total weight of the central layer 60 according to the Extractables Test Method set out herein.

Alternatively, or in addition, the central layer 60 may have an amount of titratable soluble of less than 20 %, or less than 18 %, or less than 15 %, or less than 12 %, or less than 10 %, or less than 8 %, or less than 6 %, or less than 4 % according to the Titratable Soluble Test Method set out herein.

Extractables and titratable soluble can be reduced during the manufacture of the web by subjecting it to heating (curing). Such heating may be carried out at a temperature of from 150°C to 300°C, or from 180°C to 240°C, or from 200 to 225°C, and may be carried out for 10 seconds to 30 min, or from 1 min to 15 min, or from 3 min to 10 min, or from 4 min to 8 min. The heating can be done by hot air via circulation heaters, electromagnetic irradiation (such as IR), or halogen heaters or the like.

**Supplementary absorbent layer 62**

While Fig. 2 and Fig. 3 show respectively an article comprising a single apertured central layer and a dual apertured central layer, the absorbent article may also comprise a supplementary absorbent layer 62 below the central apertured layer 60. The supplementary absorbent layer may have the same dimensions widthwise and/or lengthwise as the apertured central layer, or may have smaller or larger dimensions. A supplementary absorbent layer 62 may be advantageous when the apertured central layer 60 may have too little absorbent capacity for the intended usage.

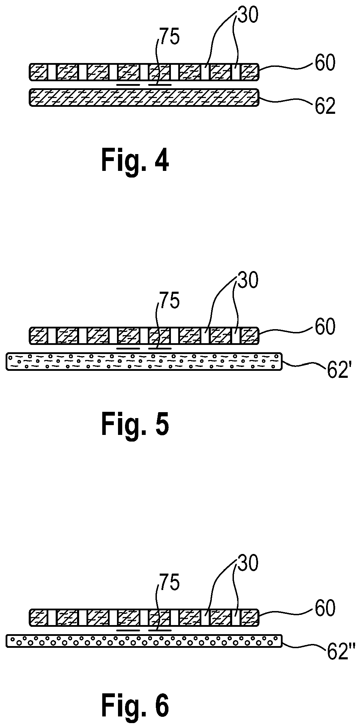

Here are some examples of non-limiting supplementary absorbent layers. As illustrated in Fig. 4, the supplementary absorbent layer 62 may be a nonwoven layer comprising or consisting of superabsorbent fibers similar to the central layer 60. In a particular example, the supplementary absorbent layer may be the same or same type of nonwoven 60 comprising or consisting of superabsorbent fibers but without the apertures discussed above for the central layer.

The supplementary absorbent layer may also be a layer 62' comprising superabsorbent particles (SAP) mixed with cellulose fibers as illustrated in Fig. 5. Similarly to SAF, SAP are water-insoluble, water-swellable polymers capable of absorbing large quantities of fluids, as is known in the art, but particles are not fiber shaped, rather SAP may assume a variety of shapes such as irregular shapes (chunks, flakes etc.), globular shapes etc. as is known in the art. SAP are typically cross-linked polymeric materials (crosslinked polyacrylc acids and their (sodium) salts), and can absorb at least 10 times their weight of an aqueous 0.9% saline solution as measured using the Centrifuge Retention Capacity (CRC) test as indicated in EDANA method NWSP 241.0.R2 (19). The SAP particles may in particular have a CRC value of more than 20 g/g, or more than 24 g/g, or of from 20 g/g to 50 g/g, or from 20 g/g to 40 g/g, or 24 g/g to 35 g/g, or 26 g/g to 32 g/g.

As illustrated in Fig. 5, the supplementary absorbent layer may also be a layer 62" comprising superabsorbent polymer (SAP) particles which are not mixed with cellulose fibers (airfelt-free layer). Such a layer of SAP particles may be immobilized on a substrate (the central layer or another layer, not represented) by a microfibrous glue, as is known in the art. The resulting layer of absorbent material may thus have a reduced thickness in the dry state, compared to conventional airfelt-based absorbent cores. The reduced thickness helps to improve the fit and comfort of the absorbent article for the wearer. The absorbent material in the supplementary absorbent layer may be free of cellulose fibers (however the absorbent core may still comprise some cellulose fibers in a nonwoven or tissue layer, such as a spunlace layer, for example as a core wrap layer).

Various absorbent core designs comprising a layer of SAP free of cellulose fibers have been proposed in the past, see for example in US5,599,335 (Goldman), EP1,447,066 (Busam), WO95/11652 (Tanzer), US2008/0312622A1 (Hundorf), WO2012/052172 (Van Malderen). In particular, the SAP printing technology as disclosed in US2006/024433 (Blessing), US2008/312,622A1, US2008/0312617A1 and US2010/0051166A1 (to Hundorf et al.) may be used.

The supplementary absorbent layer 62 may be comprised in a core wrap if needed (not represented), so that the absorbent layer can be easily integrated with the rest of the chassis of the absorbent article in a converting line. However, some absorbent material exists that do not require a core wrap, such as an airlaid absorbent core or a coform absorbent layer, and these may also be used directly in an article of the invention.

The total absorbent capacity of the absorbent article is adapted to the need of the expected wearer of the article. Diapers for newborns require typically less absorption capacity than infant or adult heavy incontinence diapers, hence less SAP and/or SAF. The absorbent article may for example comprise in the range of from about 2 g to 40 g of SAF and/or SAP in an absorbent article, in particular from 5 g to 20 g of SAF and/or SAP, for the small size diapers considered as main application of the invention. However, the invention may also be used for larger wearers, even adult wearers, where more SAF and/or SAP may be required.

**Backsheet**

The backsheet 25 is generally the portion of the absorbent article 20 that constitutes all or a part of the garment-facing side of the absorbent article. The backsheet 25 may be joined at least partially to the topsheet 24, the absorbent core 28, or other layers of the article by any attachment methods known to those of skill in the art. The backsheet prevents, or at least inhibits, the bodily exudates absorbed and contained in the absorbent core from soiling articles such as bedsheets, undergarments, and/or clothing. The backsheet is typically liquid impermeable, or at least substantially liquid impermeable.

The backsheet typically comprises a thin impermeable plastic film, usually a thermoplastic film having a thickness of about 0.01 mm to about 0.05 mm. The backsheet material may be breathable, which permit vapors to escape from the absorbent article, while still preventing, or at least inhibiting, bodily exudates from passing through the backsheet. A breathable backsheet may have a Water Vapor Transmission Rate (WVTR) of from 1,000 to 15,000 $g/m^2/24h$, or from 1,000 to 10,000 $g/m^2/24h$, or from 1,500 to 10,000 $g/m^2/24h$ as measured using a PERMATRAN-W Model 101K (available from Mocon, Inc., Minneapolis, MN) or equivalent, according to Nonwovens Standard Procedure NWSP 70.4.R0(15) with the following specifications: experiments were carried out in a lab controlled at 23 °C $\pm$ 2 C° and 50 %RH $\pm$ 2 %RH and the instrument cells heated to 37.8°C (100°F).

The backsheet 25 may also comprise a backsheet outer cover nonwoven (not represented separately in the Figures). The backsheet outer cover nonwoven is typically a thin nonwoven material that is joined to the outer surface of the backsheet film. The outer cover nonwoven may thus form the garment-facing side of the backsheet. The backsheet outer cover nonwoven may comprise a carded calendered nonwoven.

## Examples

### Apertured central layer example

Example 1: an exemplary apertured central layer is shown in Fig. 7. This central layer comprised two stacked 150 gsm SAF fiber nonwovens. The SAF fibers were sourced from Technical Absorbents, https://techabsorbents.com/, grade 112/52/10. The fibers were needled punched to form the nonwoven layers, then cured at 220°C for 4 minutes and coated with 1.75 $g/m^2$ of a surfactant solution (Hedipin® CFA-100 in a 0.5% solution). Two layers of this SAF 150 gsm nonwoven were then stacked and mechanically punched using a hydraulic clicker press to obtain circular apertures having a diameter of 5 mm. The resulting central layer had round apertures extending through the thickness of the central SAF layer. There were about 210 apertures covering about 20% of the surface of nonwoven. After aperturing, the stacked central layer had a basis weight of about 290 gsm (it is believed that not all of the SAF fibers are punched out but partially remain on the side of the apertures), a caliper of 3.04 mm at 0.85 kPa and a density of about 0.09 $g/cm^3$ at 0.85 kPa, as measured by the Caliper and Density Measurement Method disclosed hereinbelow. The individual surface area of the apertures was about 20 $mm^2$. The individual volume of the cylindrical apertures was estimated at about 60 $mm^3$ (based on the caliper measured at a pressure of 0.85 kPa). The SAF-CRC value of this first example was measured to be 24.7 g/g (1.7 g/g standard deviation).

Example 2: an alternative, exemplary apertured central layer of the invention was made from the same base stacked SAF nonwoven, but punching smaller apertures of 3 mm diameter instead of 5 mm diameter in the first example. The circular apertures having a diameter of 3 mm that extend through the thickness of the SAF nonwoven. There were about 580 apertures covering about 20% of the surface of nonwoven. The individual volumes of the cylindrical apertures are estimated to be about 11 $mm^3$ at a pressure of 0.85 kPa.

While the apertured nonwovens described above may be used as single layer in an absorbent article, in the construction examples below two layers of the same apertured SAF nonwovens were superposed to form a dual layer construction (as in Fig. 3), practically forming a central layer 60, 60' having twice the thickness of the individual layers. The caliper of the apertured dual central layer was thus of about 3 mm at 0.85 kPa. The density was about 0.09 $g/cm^3$, as measured by the Caliper and Density Measurement Method at 0.85 kPa as disclosed hereinbelow. The individual volume of the cylindrical apertures was double as for the single layer, and thus estimated to be about 60 $mm^3$ (at a pressure of 0.85 kPa). The two layers of the base SAF nonwoven can be perforated in one step by superposing the two layers on the clicker press.

### Topsheet - acquisition layer laminate example

The dual central layers described above can be combined with an acquisition layer (43 gsm PET fibers resin bonded) and a topsheet (12 gsm, non-apertured, hydrophilic, PP spunbond).

The topsheet - acquisition layer laminate had a Total Stiffness as measured with the Handle-o-meter test method of 28.8 grams.

The topsheet - acquisition layer laminate had a Horizontal Bending Drop Value of 65 mm, as measured on 5 samples according to the Horizontal Bending Drop Value test described below.

The inventive example had a three-dimensional topography as the topsheet/acquisition layer laminate could easily conform to the three-dimensionality of the central layer ("Hills and Valleys") especially when exposed to the low viscosity fecal matter.

BM rolling test

In a first testing, the dual SAF apertured central layer of example 1 as described above, an acquisition layer (43 gsm PET fibers resin bonded) and a topsheet (12 gsm, non-apertured, hydrophilic, PP spunbond) were stacked and tested against a current market product construction consisting of a patterned apertured topsheet ("PATS") and a dual layer acquisition-distribution system (as is used in Pampers® Premium Protection product).

These examples were tested in the rolling BM test. In this test, 5 g of BM-simulant (0.5% Carbopol 981 in a 0.05M NaOH solution, with 0.1 mg/mL indigo blue colorant) are uniformly applied on a 12 $cm^2$ rectangular area (20mmx60mm) on the topsheet, and then is pressed in a single forward roll with a weighted circular roll covered with filter paper and collagen attached with double sided tape, without adding any additional pressure.

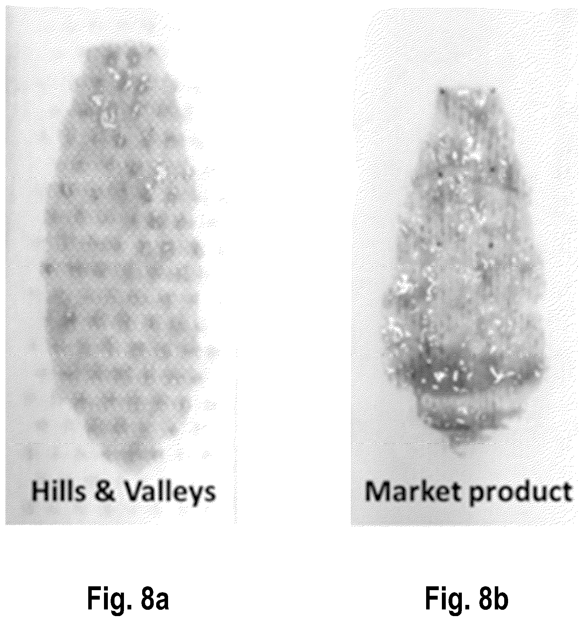

The stain size was photographed as represented in Fig. 8a and 8b and then computer analyzed. The stain size for the inventive example was slightly larger (8,286 $mm^2$ (SD=228) vs 7,071 $mm^2$ (SD=799 $mm^2$)) than the comparative example. However, as seen in the inset pictures in Fig. 8A-B, the inventive example had a more even spreading of the runny stool simulant at the surface of the topsheet.

While not wishing to be bound by theory, it is believed that the 3-dimensionality provided by the apertured central layer ("Hills & Valleys") of the invention drives a better spreading of viscous fluid (runny BM) than existing product. In the present invention, the three-dimensionality on the wearer-facing side of the article is provided by the topsheet at least partially conforming to the apertures in the central layer of the article.

Mannequin test:

A handmade inventive diaper comprising the layers described above was tested in the Mannequin test against a marketed diaper (Pampers® Premium Protection Size 2 Western Europe). The Mannequin test uses a baby mannequin on which the diaper can be fitted. 40-45 mL of BM simulant (0.5% Carbopol as indicated above) is excreted by the Mannequin in the anal region. The mannequin is sat on a roll and subsequently laid down three times as a stress test for containing BM. The process is repeated on three diapers for each example. Any leakage is collected on one or more tissues, the weight of the BM collected being calculated by weighing the soiled tissues and subtracting the weight of the tissues before usage. The diaper is then taken off and the stain size analyzed (after removing the leg cuffs) and any residue on skin is weighed using the same tissue method.

The inventive example had a smaller average stain size (6.7% smaller stain area) with the apertured SAF nonwoven central layer with a non-apertured topsheet, compared to a market product with PATS. The inventive diaper was slightly better or parity with market product for amount of BM on skin and parity for BM leaked from diaper.

Trans Topsheet Capacity (TTSC) test:

The TTSC method (Trans Topsheet Capacity) determines the BM amount passed through the topsheet and being absorbed by layers below the topsheet in a given time (e.g. 15 s) under a given pressure (about 50 kg/$m^2$) using a stainless steel hollow cylinder having an inner diameter of 5.02 cm (2 inches) and a corresponding cylindrically shaped weight. In this method, 10 mL of the BM simulant indicated above are placed in the hollow cylinder and pushed down in contact with the samples by the weight.

The samples that were prepared for this experiment were as follow:

Example A was according to the invention and comprised the same nonwoven topsheet (TS) and acquisition layer (AQL) as for the inventive example above, and two superposed 150 gsm apertured layers of SAF nonwovens (as illustrated in Fig. 3).

Example B was as example A with the differences that the two 150 gsm layers of SAF nonwovens were not apertured.

Example C was as example A with the difference that the acquisition layer was also apertured.

Example D was as example C with the difference that all the layers (topsheet, acquisition layer and SAF layers) were apertured.

Example E was a stack with a PATS topsheet an acquisition layer and an airfelt-free core.

All apertures were made with the same punch tool. In a first series, the apertures were circular with 5 mm diameter, as illustrated in Fig. 7. In a second series the apertures were circular with 3 mm diameter. The measurements were repeated 3 times for each sample.

The results were as follows (in g of Carbopol absorbed in 15 s under 50 kg/$m^2$ of pressure over a circular test area of 20.3 $cm^2$):

| Sample | BM amount passed (in g/m$^2$) |
|---|---|
| A: TS + AQL + 2 x apertured SAF | |
|    5 mm diameter apertures | 1020 (SD=100) |
|    3 mm diameter apertures | 990 (SD=130) |
| B: TS + AQL + 2 x non apertured SAF | 870 (SD=130) |
| C: TS + AQL (apertured) + 2 x SAF (apertured) | |
|    5 mm diameter apertures | 800 (SD=190) |
|    3 mm diameter apertures | 730 (SD=330) |
| D: TS + AQL + 2 X SAF (all layer apertured) | |
|    5 mm diameter apertures | 1100 (SD=70) |
|    3 mm diameter apertures | 1030 (SD=50) |
| E: PATS TS + AQL + AFF | 780 (SD=100) |

This experiment shows that perforating the AQL negatively impacted absorbency. The presence of a non-apertured AQL optimized absorption with a hydrophilic, non-apertured topsheet. This is speculated to be necessary to enable initial absorption through the topsheet.

The TTSC method also showed parity results for both 5 mm holes and 3 mm holes (the apertures both covering about 20% of the area of the central layer), suggesting a range of sizes can achieve similar results.

TEST METHODS

**Caliper and Density Measurement Method**

This method can be used to measure the caliper (thickness) of a lofty layer in a standardized manner. The density can then be calculated from the thickness and the basis weight of the layer. The measurement should preferably be made on the material before it is converted into an absorbent article. If the starting material is not available, the layer of interest (in particular the apertured central layer) can be obtained by carefully extracting it from the article. A freeze spray may be used to make the adhesive brittle and easily separate the central layer from the other layers. The samples should be kept at least 24 hours at 21°C ± 2°C in a sealed plastic bag to avoid ambient humidity being absorbed by the central layer.

Equipment: Dickenmessgerät DM 2000 from Wolf Messtechnik GmbH with a resolution of 0.01 mm, or equivalent instrument.

Contact Foot: Flat circular foot with a diameter of 40.0 mm. On its own, the foot has a weight of 109 g and exerts a pressure of 0.85 kPa.

An additional circular weight may be applied to the foot to achieve a target weight. The total weight of foot and added weight (including shaft) is selected to provide a desired pressure. The applied pressure is indicated when reporting the thickness and density, for example measured at 2.07 kPa of pressure (0.3 psi).

The caliper gauge is mounted with the lower surface of the contact foot in a horizontal plane so that the lower surface of the contact foot contacts the center of the flat horizontal upper surface of a base plate approximately 20 x 55 cm. The gauge is set to read zero with the contact foot resting on the base plate.

Stopwatch: Accuracy 1 second.

Sample preparation: The central layer is conditioned at least 24 hours as indicated above.

Measurement procedure: The layer is laid flat with the bottom side, i.e. the side intended to be placed towards the backsheet in the finished article facing down. Each sample was measured at three points, evenly spread across the sample (left, middle, right). Each point of measurement is carefully drawn on the top side of the layer, taking care not to compress or deform the layer. The values are measured at the three points of the sample.

The contact foot of the caliper gauge is raised and the layer is placed flat on the base plate of the caliper gauge with the top side of the layer up so that when lowered, the center of the foot is on the marked measuring point.

The foot is gently lowered onto the sample and released (ensure calibration to "0" prior to the start of the measurement). The caliper value is read to the nearest 0.01 mm, 5 seconds after the foot is released.

The procedure is repeated on at least 3 samples measured in this manner for a given material and the average thickness is calculated and reported with an accuracy of one tenth mm.

The basis weight of each sample is calculated by dividing the weight of each sample by their area.

The density, in g/cm$^3$, is calculated by dividing the basis weight (in g/cm$^2$) of the material by the thickness (in cm).

**Horizontal Bending Drop at 100 mm (HBD@100mm) Measurement Method**

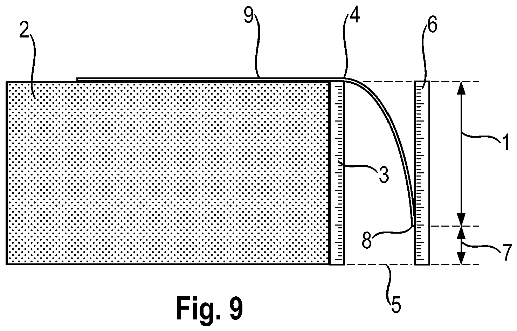

**Principle:** this method measures the ability of a nonwoven material to bend under its own weight (sometimes designated as "drapability"). The measurement principle is to hang a length of 100 mm of the material over a sharp 90° edge and measure the vertical drop of this length of the material under its own weight, expressed in mm. This vertical drop is illustrated as reference number 1 in Fig. 9.

**Apparatus:** the setup for conducting the measurement is schematically shown in Fig. 9 and comprises:

i) a flat support box 2 made of any suitable material such as polycarbonate (e.g. Lexan®) which is 400 mm long, 165 mm wide and having a height 3 of exactly 140 mm, with at least one of its top edge 4 in the width direction having a sharp 90 degree angle. The box 1 is positioned on a suitable flat surface 5, such as a lab bench;
ii) a movable vertical metal ruler 6, having a stable horizontal foot, and calibrated so that its zero corresponds to the flat surface 5 on which the box is disposed. The movable vertical metal ruler is used to measure the distance 7 of the hanging edge 8 of the material specimen 9 to the flat surface.

**Procedure:** a rectangular material specimen 9 having a width of about 80 mm and a length of about 200 mm is cut from a roll stock of the nonwoven. The length corresponds to the machine direction of the nonwoven and the width corresponds to the cross-direction of the nonwoven. The method can be alternatively conducted on a material specimen having a width of about 50 mm if the nonwoven original's width is shorter than 80 mm.

The material specimen 9 is laid flat on any suitable flat surface such as a lab bench, and a line is drawn at exactly at 100 mm from the front edge 8 of the material specimen in the width direction.

The material specimen 9 is then laid on top of the support box 2 with a first side of the specimen facing up (side A). The 100 mm line drawn is precisely positioned on the sharp edge 4 with the 100 mm long portion of the material specimen hanging free from the support box 2, as illustrated on Fig. 9. A metal ruler may be placed on the material specimen flat at the edge of the box to remove any bulging of the material at the box edge.

The movable ruler 6 is positioned near the front edge 8 of the hanging specimen material so that the distance 7 of the hanging front edge 8 from the flat surface 5 can be measured. Since the hanging front edge 8 may not be perfectly horizontal, the distance is measured on the two corners of the hanging front edge 8, as well as in the center of the front edge 8, and the arithmetic mean of the three values recorded to the nearest mm.

The bending drop 1 is calculated as the difference between the exact Drape box height 3 (140 mm) and the recorded vertical distance 7 of the front edge 8 to the flat surface 5, as measured with the ruler 6 from the flat surface 5.

The material specimen is then turned upside down (side B now up), and the same procedure described above is performed. The bending drop recorded overall for the material specimen is the greater of the side A bending drop and the side B bending drop.

The overall procedure above is repeated on five like material specimens. The arithmetic means of the bending drop values for the five like material specimens is reported to the nearest mm as the Horizontal Bending Drop at 100 mm (HBD@100mm) for the nonwoven tested.

**Handle-o-meter Test Method**

The Handle-o-meter test method covers the determination of the Total Stiffness of fabrics by measuring the force required to push a specimen into a slot of predetermined width with a metal blade working at a predetermined capacity.

The Handle-o-meter test method is carried out as indicated in ASTM D6828-02 (reapproved 2019) with the following modifications: a 90 mm x 90 mm sample is used instead of a 10 cm x 10 cm sample, the slot width is set to 20 mm instead of 10 mm.

At least 5 samples are measured and the values averaged.

The Total Stiffness of the fabric is expressed in g, based on the load max (g) determined with the Handle-o-meter (sum of 4 load max measurements as indicated in ASTM D6828-02).

**Aperture Dimensions Test**

Aperture dimensions, Effective Aperture Area, % Effective Open Area, Interaperture Distance measurements, among other measurements, are obtained from specimen images acquired using a flatbed scanner. The scanner is capable of scanning in reflectance mode at a resolution of 6400 dpi and 8 bit grayscale (a suitable scanner is an Epson Perfection V750 Pro from Epson America Inc., Long Beach CA or equivalent). The scanner is interfaced with a computer running an image analysis program (a suitable program is ImageJ v. 1.47 or equivalent, National Institute of Health, USA).

The specimen images are distance calibrated against an acquired image of a ruler certified by NIST. A steel frame is used to mount the specimen, which is then backed with a black glass tile (P/N 11-0050-30, available from HunterLab, Reston, VA) prior to acquiring the specimen image. The resulting image is then threshold, separating open aperture regions from specimen material regions, and analyzed using the image analysis program. All testing is performed in a conditioned room maintained at about 23 $\pm$ 2 °C and about 50 $\pm$ 2 % relative humidity.

Sample Preparation:

To obtain a specimen, tape an absorbent article to a rigid flat surface in a planar configuration. Any leg elastics may be cut to facilitate laying the article flat. A rectilinear steel frame (100 mm square, 1.5 mm thick with an opening 60 mm square) is used to mount the specimen. Take the steel frame and place double-sided adhesive tape on the bottom surface surrounding the interior opening. Remove the release paper of the tape, and adhere the steel frame to the apertured layer of the article. Align the frame so that it is parallel and perpendicular to a machine direction (MD) and a cross direction (CD) of the apertured layer. Using a razor blade excise the apertured layer from the overlying and underlying layers of the article around the outer perimeter of the frame. Carefully remove the specimen such that its longitudinal and lateral extension is maintained to avoid distortion of the apertures. A cryogenic spray (such as Cyto-Freeze, Control Company, Houston TX) may be used to remove the specimen from the neighboring layers if necessary. Five replicates obtained from five substantially similar articles are prepared for analysis. If the apertured layer of interest is too small to accommodate the steel frame, reduce the frame dimensions accordingly to accomplish the goals of removal of the specimen without distortion of the apertures while leaving an opening of sufficient size to allow for scanning a significant portion of the apertured layer. An apertured or patterned apertured substrate raw material is prepared for testing by extending or activating it under the same process conditions, and to the same extent, as it would be for use on the absorbent article, and then in its extended state adhering it to the steel frame as described above for testing. Condition the samples at about 23 °C $\pm$ 2 C° and about 50% $\pm$ 2% relative humidity for 2 hours prior to testing.

Image acquisition:

Place the ruler on the scanner bed, oriented parallel to sides of the scanner glass, and close the lid. Acquire a calibration image of the ruler in reflectance mode at a resolution of 6400 dpi (approximately 252 pixels per mm) and 8 bit grayscale, with the field of view corresponding to the dimensions of an interior of the steel frame. Save the calibration image as an uncompressed TIFF format file. Lift the lid and remove the ruler. After obtaining the calibration image, all specimens are scanned under the same conditions and measured based on the same calibration file. Next, place the framed specimen onto the center of the scanner bed, lying flat, with the outward facing surface of the specimen facing the scanner's glass surface. Orient the specimen so that sides of the frame are aligned parallel with and perpendicular to the sides of the scanner's glass surface, so that the resulting specimen image will have the MD vertically running from top to bottom. Place the black glass tile on top of the frame covering the specimen, close the lid and acquire a scanned image. Scan the remaining four replicates in like fashion. If necessary, crop all images to a rectangular field of view circumscribing the apertured region, and resave the files.

% Effective Open Area Calculation:

Open the calibration image file in the image analysis program and perform a linear distance calibration using the imaged ruler. This distance calibration scale will be applied to all subsequent specimen images prior to analysis. Open a specimen image in the image analysis program and set the distance scale. View the 8 bit histogram (0 to 255, with one bin per GL) and identify the gray level (GL) value for the minimum population located between the dark pixel peak of the aperture holes and the lighter pixel peak of the specimen material. Threshold the image at the minimum gray level value to generate a binary image. In the binary image the apertures appear as black, with a GL value of 255, and specimen as white, with a GL value of 0.

Using the image analysis program, analyze each of the discrete aperture regions. Measure and record all of the individual aperture areas to the nearest 0.01 mm$^2$, including partial apertures along the edges of the image. Discard any apertures with an area less than 0.3 mm$^2$ as "non-effective". Sum the remaining aperture areas (including whole and partial apertures), divide by the total area included in the image and multiply by 100. Record this value as the % Effective Open Area to the nearest 0.01%.

In like fashion, analyze the remaining four specimen images. Calculate and report the average % Effective Open Area values to the nearest 0.1% for the five replicates.

Effective Aperture Dimension Measurements:

Open the calibration image (containing the ruler) file in the image analysis program. Resize the resolution of the original image from 6400 dpi to 640 dpi (approximately 25.2 pixels per mm) using a bicubic interpolation. Perform a linear distance calibration using the imaged ruler. This distance calibration scale will be applied to all subsequent specimen images prior to analysis. Open a specimen image in the image analysis program. Resize the resolution of the original image from 6400 dpi to 640 dpi (approximately 25.2 pixels per mm) using a bicubic interpolation. Set the distance scale. View the 8 bit histogram (0 to 255, with one bin per GL) and identify the gray level (GL) value for the minimum population located between the dark pixel peak of the aperture holes and the lighter pixel peak of the specimen material. Threshold the image at the minimum gray level value to generate a binary image. In the binary image, the apertures appear as black, with a GL value of 255, and specimen as white, with a GL value of 0. Next, two morphological operations are performed on the binary image. First, a closing (a dilation operation followed by an erosion operation, iterations=1, pixel count=1), which removes stray fibers within an aperture hole. Second, an opening (an erosion operation followed by a dilation operation, iterations=1, pixel count=1), which removes isolated black pixels. Pad the edges of the image during the erosion step to ensure that black boundary pixels are maintained during the operation. Lastly, fill any remaining voids enclosed within the black aperture regions.

Using the image analysis program, analyze each of the discrete aperture regions. During the analysis exclude measurements of partial apertures along the edges of the image, so that only whole apertures are measured. Measure and record all of the individual effective aperture areas ("Effective Aperture Area"), perimeters, feret diameters (length of the apertures) along with its corresponding angle of orientation in degrees from 0 to 180, and minimum feret diameters (width of the apertures). Record the measurements for each of the individual elements areas to the nearest 0.01 mm$^2$, the perimeters and feret diameters (length and width), to the nearest 0.01 mm, and angles to the nearest 0.01 degree. Discard any apertures with an area less than 0.3 mm$^2$ as "non-effective". Record the number of remaining apertures, divide by the area of the image and record as the Aperture Density value. The angle of orientation for an aperture aligned with the MD (vertical in the image) will have an angle of 90 degrees. Apertures with a positive slope, increasing from left to right, will have an angle between zero and 90 degrees. Apertures with a negative slope, decreasing from left to right, will have an angle between 90 and 180 degrees. Using the individual aperture angles calculate an Absolute Feret Angle by subtracting 90 degrees from the original angle of orientation and taking its absolute value. In addition to these measurements, calculate an Aspect Ratio value for each individual aperture by dividing the aperture length by its width. Repeat this analysis for each of the remaining four replicate images. Calculate and report the statistical mean and standard deviation for each of the effective aperture dimension, the Absolute Feret Angle, and the Aspect Ratio measurements using all of the aperture values recorded from the replicates. Record the average of the individual Absolute Feret Angle measurements as the Average Absolute Feret Angle value. Calculate and report the % relative standard deviation (RSD) for each of the aperture dimension, the Absolute Feret Angle, and the Aspect Ratio measurements by dividing the standard deviation by the mean and multiplying by 100.

Inter-Aperture Distance Measurements:

The mean, standard deviation, median, and maximum distance between the apertures can be measured by further analyzing the binary image that was analyzed for the aperture dimension measurements. First, obtain a duplicate copy of the resized binary image following the morphological operations, and using the image analysis program, perform a Voronoi operation. This generates an image of cells bounded by lines of pixels having equal distance to the borders of the two nearest pattern apertures, where the pixel values are outputs from a Euclidian distance map (EDM) of the binary image. An EDM is generated when each interaperture pixel in the binary image is replaced with a value equal to that pixel's distance from the nearest pattern aperture. Next, remove the background zeros to enable statistical analysis of the distance values. This is accomplished by using the image calculator to divide the Voronoi cell image by itself to generate a 32-bit floating point image where all of the cell lines have a value of one, and the remaining parts of the image are identified as Not a Number (NaN). Lastly, using the image calculator, multiply this image by the original Voronoi cell image to generate a 32-bit floating point image where the distance values along the cell lines remain, and all of the zero values have been replaced with NaN. Next, convert the pixel distance values into actual inter-aperture distances by multiplying the values in the image by the pixel resolution of the image (approximately 0.04 mm per pixel), and then multiply the image again by 2 since the values represent the midpoint distance between apertures. Measure and record the mean, standard deviation, median and maximum inter-aperture distances for the image to the nearest 0.01 mm. Repeat this procedure for all replicate images. Calculate the % relative standard deviation (RSD) for the interaperture distance by dividing the standard deviation by the mean and multiplying by 100.

**Centrifuge Retention Capacity (CRC) test method**

The Capacity of superabsorbent particles (SAP) is determined according to the Centrifuge Retention Capacity (CRC) test method as set out in EDANA NWSP 241.0. R2(19).

**SAF Centrifuge Retention Capacity (SAF-CRC) test method**

This method (SAF-CRC test) is used to measure the absorbent capacity of a layer such as the central layer of the invention. The method is particular useful to measure the capacity of a web such as a nonwoven comprising superabsorbent fibers (SAF). The method is similar to EDANA NWSP 241.0.R2(19), but deviates in that the sampling (chapter 8 in EDANA NWSP 241.0.R2(19) the web or layer to be measured is as follows:

The layer is cut into pieces with approximately 5 mm as largest dimension. The cutting can e.g. be done manually with scissors. Care is taken that the fibrous structure is not majorly compressed before or during the cutting process. This ensures sufficient void space between the superabsorbent fibers, so they can be predominately wetted by the swelling medium at the entire surface area.

The sample for the measurement is taken carefully, e.g. with a lab pincet, to put it into the teabag. With a lab pincet, the fibers are carefully distributed in the teabag to avoid lumps and fiber lumps, if any, are carefully opened.

When sealing the teabag, care is taken that no material of the superabsorbent fibers, superabsorbent nonwovens and/or the superabsorbent core is in the area of the seal. This ensures a complete and sufficiently strong sealing of the teabag.

All other items of the test method are executed as set out in EDANA NWSP 241.0. R2(19).

**Amount of Extractables Test method**

This test method is used to quantify the mass fraction of soluble polymers of SAF that is extracted into physiological saline solution (0.9 weight-% NaCl in deionized water) via gravimetric analysis (hereinafter referred to as "amount of extractables"). The amount of extractables is determined via the dry weight of soluble components such as soluble polymers, oligomers and/or monomers in the extraction liquid.

Equipment:

Analytical Balance, Mettler Toledo AX 205 DR / XP 1203 S
Erlenmeyer Flask 250mL, VWR 214-1172
Tweezers
Parafilm, FILM SEALING PARAFILM® L75M W. 100MM 1, VWR 291-1212 Circular Shaker, IKA KS 501 D equipped with universal attachment AS 501.1 Buechner Funnel (porcelain, Ø110mm), VWR 511-2507
Filtering Flask (1-armed, 1000mL equipped with neck-sealing), VWR SART16606
Filter Papers (Ø110mm), Whatman 597, VWR 515-4309

Filtration cylinder:

made of transparent polycarbonate (e.g., Lexan®) and has an inner diameter of 6.00 cm (area = 28.27 cm2) with inner cylinder walls which are smooth. The bottom of the cylinder is faced with a stainless-steel screen cloth (ISO 9044 Material 1.4401, mesh size 0.038 mm, wire diameter 0.025 mm) that is bi-axially stretched to tautness prior to attachment to the bottom of the cylinder. The height of the filtration cylinder is approximately 60.5 mm.

Water Jet Pump Buerkle, BURK9668-0200
Beaker 50mL, VWR 213-0462P
40 mL Glass Vials, 30x80mm, VWR 548-0650
Pasteur pipettes, disposable 3 ml, VWR 612-1681
Sieve, Retsch 45$\mu$m Ø20cm 510-4607
Vacuum Oven, Heraeus VT 6130 P-BL
Cooling trap, Kinetics Titan Trap 8L
Vacuum Pump, Vacuubrand PC 2004 Vario

Equipment -or part of the equipment- can be replaced by equivalent equipment. Chemicals:

Use only reagents of recognized analytical grade, unless otherwise specified. Deionized Water: > 5 M$\Omega$ cm at 25°C (e.g. from Millipore purification system) Sodium Chloride ≥99.0% purity (e.g. from Merck,1.06400.1000)

Preparation of Sodium Chloride Solution (0.9 weight-%):

The accuracy of the concentration of the sodium chloride solution is important for the accuracy of the test result, as sodium chloride salt remains in the dried residuals and is subtracted (see equation 1), so the Amount of Extractables is the mass fraction of the extractable components of the superabsorbent fibers or of the nonwoven(s) comprising the superabsorbent fibers.

Procedure of preparation of 0.9 weight-% saline solution:

A clean volumetric flask with the appropriate volume (minimum volume of 1.0 L) (accuracy of at least 0.10%) for the preparation of 0.9 weight-% saline solution is filled to half of its final volume with deionized water and equipped with a clean stirring bar of suitable size. The volumetric flask is placed on a magnetic stirrer and a suitable rotation speed is chosen. The speed is suitable when a clear vortex develops in the solution and thus an adequate mixing is ensured. The required amount of sodium chloride for the preparation of 0.9 weight-% saline solution is weighed to accuracy of at least 0.11% of the amount of sodium chloride into a clean and dry weighing container (e.g. beaker) of appropriate size. The sodium chloride is added into the volumetric flask. The solution is stirred until the sodium chloride has totally dissolved. Total dissolution is achieved when no solid chemical is observed remaining in the solution.

The weighing container (e.g. beaker) that contained the sodium chloride is cleaned with deionized water which is then directly added into the flask to ensure the complete carryover of all sodium chloride. The neck of the volumetric flask is cleaned at its inner side with deionized water before filling the volumetric flask to the final volume to ensure the complete carryover of all sodium chloride into the solution. The solution is stirred well to ensure all the sodium chloride has totally dissolved. Before filling up to final volume the stirring bar is removed from the flask using a magnetic rod. The stir bar and rod are rinsed with deionized water directly into the flask to ensure the complete carryover of all material. The volumetric flask is filled with deionized water to the appropriate volume (accuracy of at least 0.10%) for the preparation of 0.9 weight-% saline solution. (In volumetric flasks, typically the defined volume of the flask is reached if the lower meniscus of the solution just touches the upper edge of the calibration line. After filling up to the final volume, the final solution is mixed well (e.g. shaken and/or stirred) in order to achieve uniform distribution of all components of the solution (to avoid any concentration gradient in the 0.9 weight-% saline solution).

Sample Preparation for Amount of Extractables:

Samples are stored in closed containers or bags, e.g. Ziplock bags, to prevent absorption of atmospheric moisture, and allowed to equilibrate to the laboratory conditions for at least 24 hours.

The superabsorbent fibers and/or nonwoven web(s) comprising superabsorbent fibers are cut into pieces with approximately 5 mm as largest dimension. The cutting can e.g. be done manually with scissors. Care is taken that the fibrous structure (i.e. the nonwoven web or the bulk of fibers) is not majorly compressed before or during the cutting process. This ensures sufficient void space between the superabsorbent fibers or in the nonwoven web(s), respectively, so they can be predominately wetted by the extraction medium at the entire surface area.

Procedure of Amount of Extractables:

1.00g ($\pm$ 0.05g) of the fibrous structure sample (superabsorbent fibers or in the nonwoven web(s) comprising superabsorbent fibers) is weighed into a 250mL Erlenmeyer flask. The mass $m1$ of the fibrous structure sample is recorded to nearest 0.001g. 200g ($\pm$1g) of 0.9 weight-% saline (prepared as described above) is added to the 250mL Erlenmeyer flask with the fibrous structure sample. The mass of the saline is recorded as $ms$ to nearest 0.001g. The Erlenmeyer flask is closed with parafilm (e.g. FILM SEALING PARAFILM® L75M W. 100MM 1, VWR 291-1212), placed onto a circular shaker (e.g. IKA KS 501 D equipped with universal attachment AS 501.1) and fixed with the universal attachment. The Erlenmeyer flask is shaken by the circular shaker at 150 rpm for 16 hours.

The filtering flask is equipped with a neck sealing and a Buechner funnel (e.g. porcelain funnel, Ø110mm, VWR 511-2507) and connected to a vacuum ejector (e.g. Water Jet Pump Buerkle, BURK9668-0200). A filter paper (e.g. Filter Papers (Ø110mm), Whatman 597, VWR 515-4309) is placed flat into the Buechner funnel. The filtration cylinder is placed on top of the filter paper, in the central position of the Buechner funnel, such that the filter paper underneath remains flat and without wrinkles or folds. Then, the filtering flask is evacuated by starting the vacuum ejector. The Erlenmeyer flask is taken from the circular shaker and the filter paper is carefully wetted with few milliliters of the extraction solution via a Pasteur pipette to ensure all perforations in the funnel are closely covered by the filter paper. The remaining extraction mixture in the Erlenmeyer flask is then added to the filtration cylinder on the Büchner funnel such that the filtration cylinder is not filled completely, i.e. up to its upper opening, at any time. After the complete extraction mixture from the Erlenmeyer flask is added to the filtration cylinder, filtration via the vacuum ejector is continued for at least approx. 2 minutes or, if filtration is not completed after 2 minutes, until no more liquid is flowing or dropping through the funnel. Afterwards, the vacuum ejector is stopped and the Buechner funnel with the filter paper and the filtration cylinder is removed from the filtration flask. A dry and clean glass vial (e.g. 40 mL Glass Vials, 30x80mm, VWR 548-0650) is weighed and the mass is recorded as $mv$ to nearest 0.0001g. 3g ($\pm$0.0500g) of the filtrate (filtered extraction solution in the filtration flask) recorded as mass $ml$ to nearest 0.0001g, is added into the glass vial. The glass vial is covered with a screen (e.g. Sieve, Retsch 45$\mu$m Ø20cm 510-4607) to avoid any loss of solid or solution during drying.

The filtrate in the glass vial covered with the screen is dried in a vacuum oven at 40°C at <10 mbar for at least 24 hours. The drying is continued till the sample is fully dried (till the mass remains constant), but not longer than 96 hours.

After drying, the sample is weighed in the glass vial and the mass m4 is recorded to nearest 0.0001g, giving the dry weight of glass vial with dried extract (i.e. the residual of the liquid phase). In case any solid is attached to the screen, it is transferred into the vial before the mass m4 is determined. Alternatively, the mass of the screen before and after drying is determined and the difference is added to the mass of the vial with the dried extract to give m4. Note: In case the difference in screen weight before and after drying is negative, it is considered to be 0 (zero).

For each superabsorbent fiber sample or nonwoven web(s) sample comprising superabsorbent fibers, typically 4 extractions are done. For each extraction, at least 2 dryings are done. This results in typically 8 glass vials with dried extract, called herein 8 "replicates". The amount of extractables is determined based on at least 4 replicates, typically based on 8 replicates.

Calculation:

Amount of extractables (weight-%) via Extraction Liquid

Extracted Amount of solid (g) = me

$$m_e = m_4 - m_v - 0.009 \cdot m_l \quad (1)$$

Extractables = extr.

$$extr. = (m_s \cdot m_e)/((m_l - m_e) \cdot m_1) \quad (2)$$

The mean of all replicates of "extr." is calculated and given as "amount of extractables" in weight-%.

In case a replicate shows a negative value for "extr." (this can occur for very small amount of extractables as the dry sodium chloride amount is subtracted from the dried solid (see equation 1), this replicate is discarded. In case at least 4 replicates of the respective superabsorbent fiber sample or nonwoven web(s) sample comprising superabsorbent fibers show positive values for "extr.", the average of these replicates is taken to give amount of extractables" in weight-%.

In case less than 4 replicates (out of the 8 replicates done) of the respective superabsorbent fiber sample or nonwoven web(s) sample comprising superabsorbent fibers show positive values for "extr.", "amount of extractable" for that sample is defined as <10 weight-%.

**Titratable Soluble Test method**

This test method is used to quantify titratable soluble after extraction of superabsorbent fiber sample or nonwoven web(s) sample comprising superabsorbent fibers with 0.9 weight-% saline.

Preparation of 0.9 weight-% saline as described under Test Method "Amount of Extractables" herein.

Sample Preparation for titratable soluble:
Samples are stored in closed containers or bags, e.g. Ziplock bags, to prevent absorption of atmospheric moisture, and allowed to equilibrate to the laboratory conditions for at least 24 hours.

The superabsorbent fibers and/or nonwoven web(s) comprising superabsorbent fibers are cut into pieces with approximately 5 mm as largest dimension. The cutting can e.g. be done manually with scissors. Care is taken that the fibrous structure (i.e. the nonwoven web or the bulk of fibers) is not majorly compressed before or during the cutting process. This ensures sufficient void space between the superabsorbent fibers or in the nonwoven web(s), respectively, so they can be predominately wetted by the extraction medium at the entire surface area.

Procedure of titratable soluble:
The test method titratable soluble is executed according to the procedure described in EDANA NWSP 270.0.R2 (19) with extraction time of 16 hours, instead of 1 hour and except the sampling (chapter 8 of EDANA NWSP 270.0.R2 (19), "Sampling"). The sample preparation for titratable soluble is done as described above.

The calculation of titratable soluble is done according to the calculation of the extractable content, w, in chapter 10 "Calculation" of EDANA NWSP 270.0.R2 (19). Titratable soluble is given in %.

Notably, titratable soluble may not represent a mass fraction, as the molecular weight of the monomeric components in the superabsorbent fibers and/or nonwoven web(s) comprising superabsorbent fibers might be different to the molecular weights used in EDANA NWSP 270.0.R2 (19) in chapter 10, MCOOH and MCOONa.

**EP 4 643 832 A1**

<u>Misc</u>

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

Unless indicated otherwise, the description and claims refer to the absorbent core and article before use (i.e. dry, and not loaded with a fluid) and conditioned at least 24 hours at 21 °C +/- 2 °C and 50 +/- 5% Relative Humidity (RH).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

**Claims**

1. A personal absorbent article (20) comprising in this order a topsheet (24), an optional acquisition layer (50), an apertured central layer (60) comprising a plurality of apertures (30), and a backsheet (25), wherein the topsheet, together with the optional acquisition layer when present, can at least partially conform to the apertures of the central layer so that a three-dimensional surface on the wearer-facing side of the article is provided during use.

2. The article according to claim 1, wherein the topsheet is a non-apertured nonwoven.

3. The article according to claim 1 or 2, wherein the apertured central layer has a % Effective Open Area in the range of from 10% to 50%, as measured on the topsheet-facing side of the central layer by the Aperture Dimensions Test as described herein.

4. The article according to any of the preceding claims, wherein the apertures of the central layer have an Effective Aperture Area in the range of from 2 $mm^2$ to 200 $mm^2$, in particular of from 3 $mm^2$ to 80 $mm^2$, as measured on the topsheet-facing side of the central layer by the Aperture Dimensions Test as described herein; and/or wherein the central layer (60, 60') has a caliper at 0.85 kPa of at least 1 mm, in particular from 2 mm to 20 mm, as measured by Caliper and Density Measurement Method disclosed herein.

5. The article according to any of the preceding claims, wherein the apertures have an average individual volume in the range of from 10 $mm^3$ to 2,000 $mm^3$, wherein the average individual volume is calculated by multiplying the Effective Aperture Area by the caliper of the central layer, wherein the caliper is measured at a pressure 0.85 kPa according to the Caliper and Density Measurement Method disclosed herein.

6. The article according to any of the preceding claims, wherein the central absorbent layer comprises from 10 to 10,000 apertures, in particular from 50 to 2,000 apertures, in particular from 100 to 1,000 apertures.

7. The article according to any of the preceding claims, wherein the central layer (60, 60') has density in the range of from 0.01 $g/cm^3$ to 0.5 $g/cm^3$ as measured at a pressure of 0.85 kPa by the Caliper and Density Measurement Method disclosed herein.

8. The article according to any of the preceding claims, wherein the central layer is a nonwoven comprising or consisting of superabsorbent fibers, preferably wherein the central layer has at least 7 g/g SAF Centrifuge Retention Capacity (SAF-CRC) as measured by the SAF-CRC test described herein.

9. The article according to any of the preceding claims, wherein the topsheet, together with the acquisition layer when present, has a Horizontal Bending Drop Value of at least 55 mm, as measured by the Horizontal Bending Drop Test disclosed herein.

10. The article according to any of the preceding claims, wherein the topsheet, together with the acquisition layer when present, has a Total Stiffness under 90 g as measured by the Handle-o-meter test method, in particular wherein the Total Stiffness is in the range of from 10 g to 80 g.

11. A personal absorbent article (20) comprising in this order a topsheet (24), an acquisition layer (50), an apertured central layer (60) comprising a plurality of apertures and a backsheet (25),

wherein the topsheet is a non-apertured nonwoven;
wherein the acquisition layer is a non-apertured nonwoven having a basis weight in the range of from 20 gsm to 80 gsm;
wherein the topsheet and the acquisition layer form a laminate by at least partially bonding together the topsheet and acquisition layer, for example by adhesive bonding;
wherein the apertures of the central layer have an Effective Aperture Area in the range of from $2 \, mm^2$ to $500 \, mm^2$, preferably of from $3 \, mm^2$ to $300 \, mm^2$, as measured on the topsheet-facing side of the central layer by the Aperture Dimensions Test as described herein;
wherein the topsheet-acquisition layer laminate has a Horizontal Bending Drop Value of at least 55 mm, as measured by the Horizontal Bending Drop Test disclosed herein, and/or a has a Total Stiffness under 90 g, as measured by the Handle-o-meter test method described herein.

12. A personal absorbent article (20) comprising in this order a topsheet (24), an optional acquisition layer (50), an apertured central layer (60, 60') and a backsheet (25), wherein the apertured central layer (60, 60') comprises from about 20% to about 100% of superabsorbent fibers (SAF) by weight of the apertured central layer.

13. A personal absorbent article (20) comprising in this order a topsheet (24), an optional acquisition layer (50), an apertured central layer (60, 60') and a backsheet (25), wherein the apertured central layer (60, 60') is a nonwoven comprising or consisting of superabsorbent fibers, and wherein the apertured central layer has at least 7 g/g SAF Centrifuge Retention Capacity (SAF-CRC) as measured by the SAF-CRC test described herein.

14. A personal absorbent article (20) comprising in this order a topsheet (24), an acquisition layer (50), an apertured central layer (60) comprising a plurality of apertures, and a backsheet (25),

wherein the topsheet is a non-apertured nonwoven;
wherein the topsheet and the acquisition layer form a laminate by at least partially bonding together the topsheet and acquisition layer, for example by adhesive bonding;
wherein the apertured central layer is a nonwoven comprising or consisting of superabsorbent fibers;
wherein the apertures have an average individual volume in the range of from $10 \, mm^3$ to $2,000 \, mm^3$, wherein the average individual volume is calculated by multiplying the Effective Aperture Area by the caliper of the central layer, wherein the caliper is measured at a pressure 0.85 kPa according to the Caliper and Density Measurement Method disclosed herein; and
wherein the topsheet-acquisition layer laminate has a Horizontal Bending Drop Value of at least 55 mm, as measured by the Horizontal Bending Drop Test disclosed herein and/or a has a Total Stiffness under 100 g, as measured by the Handle-o-meter test method as described herein.

15. A method for making a personal absorbent article according to any of the preceding claims, comprising the step of forming the apertures in the apertured central layer by a slit-and-stretch method.

# Fig. 1

**Fig. 2**

**Fig. 3**

Fig. 4

Fig. 5

Fig. 6

Fig. 7

Hills & Valleys

Fig. 8a

Market product

Fig. 8b

**Fig. 9**

Europäisches
Patentamt

European
Patent Office

Office européen
des brevets

# EUROPEAN SEARCH REPORT

**Application Number**

EP 24 17 3166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/258649 A1 (ROSATI RODRIGO [DE] ET AL) 14 September 2017 (2017-09-14) | 1,2,4-7 | INV.<br>A61F13/15 |
| Y | * paragraphs [0004], [0081] - [0084], [0104], [0154]; claim 1; figures 25-28 * | 3,8-15 | A61F13/494<br>A61F13/511<br>A61F13/512 |
| Y | WO 2016/073712 A1 (PROCTER & GAMBLE [US]) 12 May 2016 (2016-05-12)<br>* claims 1-15 * | 3 | A61F13/53 |
| Y | WO 2007/034453 A1 (PROCTER & GAMBLE [US]; PONOMARENKO EKATARINA ANATOLYE [DE] ET AL.) 29 March 2007 (2007-03-29)<br>* page 6, lines 10-14; claims 1-8 * | 8 | |
| Y | US 2020/306105 A1 (PERI ANDREA [DE] ET AL) 1 October 2020 (2020-10-01)<br>* paragraphs [0055], [0118]; claims 1-17 * | 9-14 | |
| Y | US 11 628 100 B2 (PROCTER & GAMBLE [US]) 18 April 2023 (2023-04-18)<br>* column 18, lines 4-53 * | 15 | |

**TECHNICAL FIELDS SEARCHED (IPC)**

A61F

The present search report has been drawn up for all claims

| Place of search | Date of completion of the search | Examiner |
|---|---|---|
| Munich | 10 October 2024 | Adechy, Miriam |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.........................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 17 3166

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2024

| Patent document cited in search report | Publication date | Patent family member(s) | | Publication date |
|---|---|---|---|---|
| US 2017258649 A1 | 14-09-2017 | US | 2017258645 A1 | 14-09-2017 |
| | | US | 2017258648 A1 | 14-09-2017 |
| | | US | 2017258649 A1 | 14-09-2017 |
| | | WO | 2017156196 A1 | 14-09-2017 |
| | | WO | 2017156197 A1 | 14-09-2017 |
| | | WO | 2017156200 A1 | 14-09-2017 |
| WO 2016073712 A1 | 12-05-2016 | BR | 112017009580 A2 | 26-12-2017 |
| | | BR | 112017009636 A2 | 19-12-2017 |
| | | CA | 2967001 A1 | 12-05-2016 |
| | | CA | 2967014 A1 | 12-05-2016 |
| | | CL | 2017001114 A1 | 26-01-2018 |
| | | CN | 107072835 A | 18-08-2017 |
| | | CN | 107072836 A | 18-08-2017 |
| | | CN | 107072849 A | 18-08-2017 |
| | | CN | 107072850 A | 18-08-2017 |
| | | CN | 107106339 A | 29-08-2017 |
| | | CN | 107106340 A | 29-08-2017 |
| | | CN | 107106341 A | 29-08-2017 |
| | | CN | 107106342 A | 29-08-2017 |
| | | CN | 107249534 A | 13-10-2017 |
| | | CN | 107249535 A | 13-10-2017 |
| | | EP | 3215084 A1 | 13-09-2017 |
| | | EP | 3215087 A1 | 13-09-2017 |
| | | EP | 3215089 A1 | 13-09-2017 |
| | | EP | 3215090 A1 | 13-09-2017 |
| | | EP | 3215091 A1 | 13-09-2017 |
| | | EP | 3215092 A1 | 13-09-2017 |
| | | EP | 3215093 A1 | 13-09-2017 |
| | | EP | 3215094 A1 | 13-09-2017 |
| | | EP | 3215095 A1 | 13-09-2017 |
| | | EP | 3215096 A1 | 13-09-2017 |
| | | EP | 4268779 A2 | 01-11-2023 |
| | | JP | 6976850 B2 | 08-12-2021 |
| | | JP | 2017533021 A | 09-11-2017 |
| | | JP | 2017533044 A | 09-11-2017 |
| | | JP | 2017535333 A | 30-11-2017 |
| | | JP | 2017535452 A | 30-11-2017 |
| | | JP | 2017535453 A | 30-11-2017 |
| | | JP | 2017536874 A | 14-12-2017 |
| | | JP | 2017536882 A | 14-12-2017 |
| | | JP | 2017537677 A | 21-12-2017 |
| | | JP | 2017537680 A | 21-12-2017 |
| | | JP | 2019088857 A | 13-06-2019 |
| | | RU | 2017111494 A | 12-12-2018 |
| | | RU | 2017112525 A | 06-12-2018 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 24 17 3166

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2024

| Patent document cited in search report | Publication date | Patent family member(s) | | Publication date |
|---|---|---|---|---|
| | | RU | 2017115217 A | 06-12-2018 |
| | | RU | 2017115220 A | 06-12-2018 |
| | | US | 2016129626 A1 | 12-05-2016 |
| | | US | 2016129661 A1 | 12-05-2016 |
| | | US | 2016129662 A1 | 12-05-2016 |
| | | US | 2016129663 A1 | 12-05-2016 |
| | | US | 2016136003 A1 | 19-05-2016 |
| | | US | 2016136010 A1 | 19-05-2016 |
| | | US | 2016136014 A1 | 19-05-2016 |
| | | US | 2016136015 A1 | 19-05-2016 |
| | | US | 2016136016 A1 | 19-05-2016 |
| | | US | 2016136919 A1 | 19-05-2016 |
| | | US | 2018000654 A1 | 04-01-2018 |
| | | US | 2018000655 A1 | 04-01-2018 |
| | | US | 2018000656 A1 | 04-01-2018 |
| | | US | 2018228660 A1 | 16-08-2018 |
| | | US | 2018235817 A1 | 23-08-2018 |
| | | US | 2018318144 A1 | 08-11-2018 |
| | | US | 2019076305 A1 | 14-03-2019 |
| | | US | 2019209398 A1 | 11-07-2019 |
| | | US | 2019240082 A1 | 08-08-2019 |
| | | US | 2019290503 A1 | 26-09-2019 |
| | | US | 2020246199 A1 | 06-08-2020 |
| | | US | 2020383843 A1 | 10-12-2020 |
| | | US | 2021322231 A1 | 21-10-2021 |
| | | US | 2022226168 A1 | 21-07-2022 |
| | | US | 2022265486 A1 | 25-08-2022 |
| | | US | 2023293361 A1 | 21-09-2023 |
| | | US | 2024074922 A1 | 07-03-2024 |
| | | US | 2024082075 A1 | 14-03-2024 |
| | | US | 2024099908 A1 | 28-03-2024 |
| | | US | 2024099909 A1 | 28-03-2024 |
| | | US | 2024108517 A1 | 04-04-2024 |
| | | WO | 2016073686 A1 | 12-05-2016 |
| | | WO | 2016073687 A1 | 12-05-2016 |
| | | WO | 2016073688 A1 | 12-05-2016 |
| | | WO | 2016073689 A1 | 12-05-2016 |
| | | WO | 2016073712 A1 | 12-05-2016 |
| | | WO | 2016073715 A1 | 12-05-2016 |
| | | WO | 2016073719 A1 | 12-05-2016 |
| | | WO | 2016073722 A1 | 12-05-2016 |
| | | WO | 2016073727 A1 | 12-05-2016 |
| | | WO | 2016073736 A1 | 12-05-2016 |
| WO 2007034453 A1 | 29-03-2007 | CA | 2625411 A1 | 29-03-2007 |
| | | CN | 101272755 A | 24-09-2008 |

EPO FORM P0459

For more details about this annex : see Official Journal of the European Patent Office, No. 12/82

page 2 of 3

## ANNEX TO THE EUROPEAN SEARCH REPORT
## ON EUROPEAN PATENT APPLICATION NO.

EP 24 17 3166

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2024

| Patent document cited in search report | | Publication date | Patent family member(s) | | Publication date |
|---|---|---|---|---|---|
| | | | EP | 1767177 A1 | 28-03-2007 |
| | | | JP | 2009508621 A | 05-03-2009 |
| | | | US | 2010228215 A1 | 09-09-2010 |
| | | | WO | 2007034453 A1 | 29-03-2007 |
| US 2020306105 | A1 | 01-10-2020 | CN | 113573680 A | 29-10-2021 |
| | | | EP | 3946197 A2 | 09-02-2022 |
| | | | US | 2020306096 A1 | 01-10-2020 |
| | | | US | 2020306105 A1 | 01-10-2020 |
| | | | WO | 2020205485 A2 | 08-10-2020 |
| US 11628100 | B2 | 18-04-2023 | EP | 3560465 A1 | 30-10-2019 |
| | | | US | 2019328588 A1 | 31-10-2019 |
| | | | WO | 2019209470 A1 | 31-10-2019 |

EPO FORM P0459

For more details about this annex : see Official Journal of the European Patent Office, No. 12/82

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170258649 A1, Rosati
- WO 2016073712 A1, Arora
- WO 2007034453 A1, Ponomarenko
- US 3860003 A
- US 5221274 A
- US 5554145 A
- US 5569234 A
- US 5580411 A
- US 6004306 A
- WO 2020205485 A, Peri
- US 3983095 A
- US 3954721 A
- US 5026784 A
- US 5151465 A
- US 4880868 A
- US 4743244 A
- US 4616063 A
- US 4705773 A
- US 4731067 A
- US 4788237 A
- US 4813945 A
- US 5280079 A
- US 5147956 A
- US 4980434 A
- US 4962172 A
- US 4997714 A
- US 4861539 A
- WO 2016191439 A, Jackels
- US 20220354713 A1, Ehrnsperger
- US 5599335 A, Goldman
- EP 1447066 A, Busam
- WO 9511652 A, Tanzer
- US 20080312622 A1, Hundorf
- WO 2012052172 A, Van Malderen
- US 2006024433 A, Blessing
- US 2008312622 A1
- US 20080312617 A1
- US 20100051166 A1, Hundorf